# DEMANDE DE BREVET EUROPEEN

(11) **EP 4 461 275 A1**
(43) Date de publication de la demande: **13.11.2024**
(21) Numéro de dépôt: 24174441.6
(22) Date de dépôt: 07.05.2024
(51) Int. Cl.: A61F 7/00, A61F 7/02, A41D 13/002, A41D 13/005, A62B 17/00, A62B 9/02, A62B 9/00

(54) **DISPOSITIF DE CHAUFFAGE CORPOREL UTILISANT L'AIR EXPIRE**

(30) Priorité: 12.05.2023 FR 2304702
(71) Demandeur: Flores, Mathieu, 25360 Bouclans (FR)
(72) Inventeur: FLORES, Mathieu, 25360 BOUCLANS (FR)
(74) Mandataire: Oudin, Stéphane

(57) **Abrégé**

Dispositif de chauffage corporel 1 utilisant l'air expiré d'un utilisateur, comportant au moins :
- un boitier 2 délimitant une chambre 3 avec un système de clapets SC comprenant au moins trois orifices 4, 5, 6 connectant l'extérieur du boitier 2 à ladite chambre 3, à savoir un orifice 4 d'inspiration, un orifice 5 de respiration et un orifice 6 d'expiration, ledit système de clapets SC comprenant un premier clapet 7 et un deuxième clapet 8 qui permettent la circulation d'air respectivement de l'orifice d'inspiration 4 vers l'orifice de respiration 5, et de l'orifice de respiration 5 vers l'orifice d'expiration 6,
- un tube de sortie 8 connecté à l'orifice d'expiration 6 et à un organe de diffusion 10 de la chaleur,
le premier clapet 7 et le deuxième clapet 8 étant formées d'une seule pièce 11 au moins partiellement élastiquement déformable.

Vêtement comportant un tel dispositif 1.

## Description

Le domaine technique concerne celui des systèmes de chauffage permettant de se réchauffer avec de l'air chaud expiré.

Le domaine de cette invention peut s'appliquer plus particulièrement au domaine des systèmes de chauffage pouvant être utilisé pour réchauffer l'air dans un manteau, un vêtement ou tout élément recouvrant tout ou partie du corps d'une personne.

Dans un environnement froid, il est connu de se réchauffer les mains en soufflant dessus lentement. L'air qu'une personne inspire est réchauffée dans les poumon, et ressort plus chaud lorsqu'il est expiré, habituellement entre 33 et 37 degrés Celsius, en fonction de la fréquence ventilatoire et de la température extérieure soit un delta avec l'air extérieur de plus de 40° à -10°C, 30° à 0°C etc...

Une personne avec un vêtement comportant un col relevé peut positionner son visage de manière à avoir le nez et la bouche au-dessus du col, puis positionner la bouche dans le col du vêtement. En inspirant par le nez ou la bouche hors du col et en soufflant par la bouche dans le col, une personne peut alors expirer de l'air réchauffé dans ses poumons dans l'espace compris entre son corps et son vêtement, lui permettant d'augmenter la température de l'air intercalé dans l'espace entre son corps et son vêtement pour se réchauffer.

L'air expiré se déploie dans cet espace surtout au niveau du cou. Or le froid se ressent plutôt au niveau du torse.

Pour y remédier, il a été imaginé un dispositif tel que décrit dans le document US5029572. Ce dispositif comporte un réseau de canalisations pouvant être porté sous un vêtement par un utilisateur. Le dispositif comporte un embout pouvant être mis en bouche par ledit utilisateur équipé dudit dispositif, permettant à l'utilisateur d'expirer de l'air chaud de ses poumons pour le faire circuler dans les canalisations du dispositif, afin de réchauffer ledit utilisateur.

Des réservoirs connectés au réseau de canalisations permettent de récupérer la condensation de l'humidité de l'air expiré.

Ce dispositif est particulièrement encombrant et inesthétique. Il gêne et/ou limite les mouvements au niveau du cou de l'utilisateur, et est au contact et provoque des frottements avec le cou et la bouche de l'utilisateur lorsqu'il ne l'utilise pas.

De plus ce dispositif nécessite un nombre important de pièces ce qui le rend couteux, et il ne peut pas être facilement installé et retiré.

En outre, il oblige l'utilisateur à inspirer par le nez et à souffler par la bouche, ce qui peut handicaper une respiration normale, surtout dans un environnement froid.

Ce dispositif n'est de plus pas adapté à un usage ponctuel, pendant quelques minutes seulement.

Une solution partielle à ces inconvénients est présente dans le document US 10617896 qui décrit un dispositif de prévention de l'hypothermie comportant un système de soupape à trois orifices ayant un orifice d'inspiration, un orifice d'expiration et un orifice de respiration apte à être collé à la bouche d'un utilisateur, dans lequel une soupape d'admission relie l'orifice d'inspiration à l'orifice de respiration, et une soupape de sortie relie l'orifice de respiration à l'orifice d'expiration, de sorte que l'air expiré est dirigé à travers l'orifice d'expiration par un tube de sortie vers des parties du corps à réchauffer de l'utilisateur.

Ce dispositif est en peu de pièces, et compact, facile à transporter et à installer pour l'utiliser afin d'éviter une hypothermie.

Ce dispositif est cependant toujours encombrant, voyant et inesthétique pour un usage dans un environnement non extrême, avec un vêtement, pour se réchauffer de manière ponctuelle. Ce dispositif gêne et/ou limite toujours les mouvements au niveau du cou de l'utilisateur, et est toujours au contact et provoque des frottements avec le cou et la bouche de l'utilisateur lorsqu'il ne l'utilise pas

C'est donc un objectif de l'invention que de proposer un dispositif de chauffage corporel utilisant l'air expiré qui soit, peu voyant, et qui puisse s'intégrer dans l'esthétisme d'un vêtement.

C'est également un objectif de l'invention que de proposer un dispositif qui soit simple à fabriquer et à mettre en oeuvre.

C'est également un objectif de l'invention que de proposer un dispositif compact permettant de respirer normalement, sans gêne.

**A cet effet l'invention concerne** un dispositif de chauffage corporel utilisant l'air expiré d'un utilisateur équipé d'un tel dispositif, ledit dispositif comportant au moins :
- un boitier délimitant une chambre avec un système de clapets comprenant au moins trois orifices connectant l'extérieur dudit boitier à ladite chambre, lesdits au moins trois orifices comportant un premier orifice dit d'inspiration, un deuxième orifice dit de respiration et un troisième orifice dit d'expiration, ledit système de clapets comprenant un premier clapet qui permet la circulation d'air de l'orifice d'inspiration vers l'orifice de respiration, et un deuxième clapet qui permet la circulation d'air de l'orifice de respiration vers l'orifice d'expiration,
- un tube de sortie connecté d'une part au troisième orifice d'expiration et d'autre part à un organe de diffusion de la chaleur.

**Avantageusement,** le premier clapet et le deuxième clapet sont formés d'une seule pièce au moins partiellement élastiquement déformable.

Cette pièce unique au moins partiellement élastiquement déformable présente au moins deux parties mobiles au moins partiellement formant respectivement le premier clapet antiretour et le deuxième clapet antiretour en appui contre des parois ajourées du boitier. Cela permet de dissocier simplement les flux lors de l'inspiration et de l'expiration, pour diffuser l'air expiré par un organe de diffusion de la chaleur. En l'absence de contraintes, ces parties formant clapets sont en appui contre les parois ajourées et viennent recouvrir et obturer les jours dans les parois.

Cette structure est simple, compacte et plate, ce qui permet de rendre compact, robuste et plat ce dispositif qui ne comporte qu'un nombre de pièces réduit.

Ce dispositif plat et compact peut discrètement être intégré dans un col d'un vêtement ou rapporté.

**Selon une caractéristique de l'invention,** la pièce au moins partiellement élastiquement déformable est une feuille en un matériau élastomère, par exemple sous la forme d'un disque en un matériau élastomère.

Sa fabrication est simple et peu coûteuse. Une feuille présente l'avantage de simplifier le montage et le sens de positionnement dans le dispositif. Dans le cas d'une forme d'un disque, cela permet de ne pas avoir à se préoccuper d'indexer ladite pièce, ce qui simplifie encore le montage.

**Selon une caractéristique de l'invention, le boitier du dispositif comporte deux parois ajourées contre lesquelles des parties de la pièce formant le premier et le deuxième clapets viennent en appui.**

Cette structure est simple, compacte et plate, ce qui permet de rendre compact, robuste et plat ce dispositif qui ne comporte qu'un nombre de pièces réduit.

**Selon une caractéristique de l'invention, l'ensemble formé du boitier avec la chambre et du système de clapets est constitué de trois éléments.**

Cela permet de réduire le nombre d'éléments constitutifs de ce dispositif.

**Selon une caractéristique de l'invention, les trois éléments constituant l'ensemble formé du boitier avec la chambre et du système de clapets sont constitués d'une part de deux demi-coques coopérant l'une avec l'autre par emboîtement, et d'autre part de la pièce formant le premier et le deuxième clapet intercalée entre les deux demi-coques.**

La fabrication du dispositif s'en voit simplifiée.

**Selon une caractéristique de l'invention, l'ensemble formé du boitier avec la chambre et du système de clapets est inclus dans un cylindre droit de 33 millimètres de diamètre maximum et 17 millimètres de hauteur maximum.**

L'ensemble formé du boitier avec la chambre et du système de clapets de par sa configuration est plus discret et plus compact que les solutions de l'art antérieur, et peut être intégré dans un col de vêtement.

**Selon une caractéristique de l'invention, la section de passage d'air entre l'orifice d'inspiration et l'orifice de respiration, et la section de passage d'air entre l'orifice de respiration et l'organe de diffusion est supérieure à 75 millimètres carrés.**

Ces dimensions minimales permettent à un utilisateur de respirer à travers le dispositif sans gêne. Ces dimensions correspondent à une section correspondant à un passage de 10 millimètres de diamètre.

En fait, malgré son caractère compact et discret lui permettant une intégration dans un vêtement de par sa construction particulière, le dispositif présente une section de passage d'air permettant à un utilisateur de respirer confortablement.

**Selon une caractéristique de l'invention, l'organe de diffusion connecté au tube de sortie est un cylindre présentant une pluralité de trous latéraux.**

Ces trous latéraux sont réalisés sur toute la périphérie, de manière à éviter qu'ils soient tous bouchés du fait d'un contact avec le vêtement équipé du dispositif, d'autres vêtement ou du corps de l'utilisateur. En effet, de par cette configuration, il existe toujours des trous qui ne sont pas couverts.

**Selon une caractéristique de l'invention, le dispositif comporte un anneau d'assemblage sur un col de vêtement, ledit anneau comportant une ouverture centrale configurée pour recevoir le boîtier.**

Ainsi, il est possible de monter un tel anneau sur un col de vêtement, ce qui permet de faciliter le montage ou démontage du dispositif sur un vêtement.

Ainsi, le dispositif ne gêne et/ou ne limite pas les mouvements au niveau du cou, et il n'y a pas de contact et de frottement sur le cou et la bouche lorsque le dispositif n'est pas utilisé. En outre le vêtement peut être utilisé avec ou sans le dispositif.

Le dispositif de par sa configuration est configuré pour une installation sur un vêtement déjà existant et ne comportant pas initialement d'ouverture pour recevoir le dispositif étant donné la finesse du boitier dudit dispositif.

En outre, étant donné ses dimensions, le boitier du dispositif peut être facilement camouflé ou réintégrer dans le col du vêtement lorsqu'il n'est pas utilisé.

**L'invention concerne également un vêtement** comportant un tel dispositif, ledit vêtement comportant également des moyens de maintien du tube de sortie et/ou de l'organe de diffusion.

D'autre buts et avantages de la présente invention apparaîtront au cours de la description qui va suivre se référant aux dessins ci-joints illustrant des exemples de réalisation et dans lesquels :
[Fig 1] est une vue schématique en perspective arrière droit d'un dispositif selon un mode de réalisation de l'invention.
[Fig 2] est une vue schématique en perspective arrière droite éclatée du dispositif de la figure 1.
[Fig 3] est une vue schématique en coupe latérale d'un boitier du dispositif de la figure 1 seul.
[Fig 4] est une vue schématique en coupe latérale d'un boitier d'un dispositif selon l'invention selon un autre mode de réalisation de l'invention en position de repos.
[Fig 5] est une vue schématique en coupe latérale d'un boitier d'un dispositif selon l'invention de la figure 4, lors d'une inspiration d'un utilisateur.
[Fig 6] est une vue schématique en coupe latérale d'un boitier d'un dispositif selon l'invention de la figure 4, lors d'une expiration d'un utilisateur.
[Fig 7] est une vue avant de l'extérieur d'un col de vêtement comportant un dispositif selon encore un autre mode de réalisation de l'invention.
[Fig 8] est une vue de l'intérieur d'un col avant du vêtement de la figure 7 comportant ledit dispositif de la figure 7.

L'invention concerne un dispositif de chauffage corporel 1 selon l'invention, désigné ci-après sous le terme « dispositif ».

Ce dispositif 1 permet à un utilisateur équipé d'un tel dispositif 1 de se réchauffer avec de l'air chaud expiré en l'envoyant vers au moins une partie de son corps.

Le dispositif 1 comporte au moins un boitier 2 délimitant une chambre 3.

Ce boitier 2 peut présenter une forme générale cylindrique, selon une direction longitudinale arrière-avant, avec une paroi arrière et une paroi avant en forme de disque.

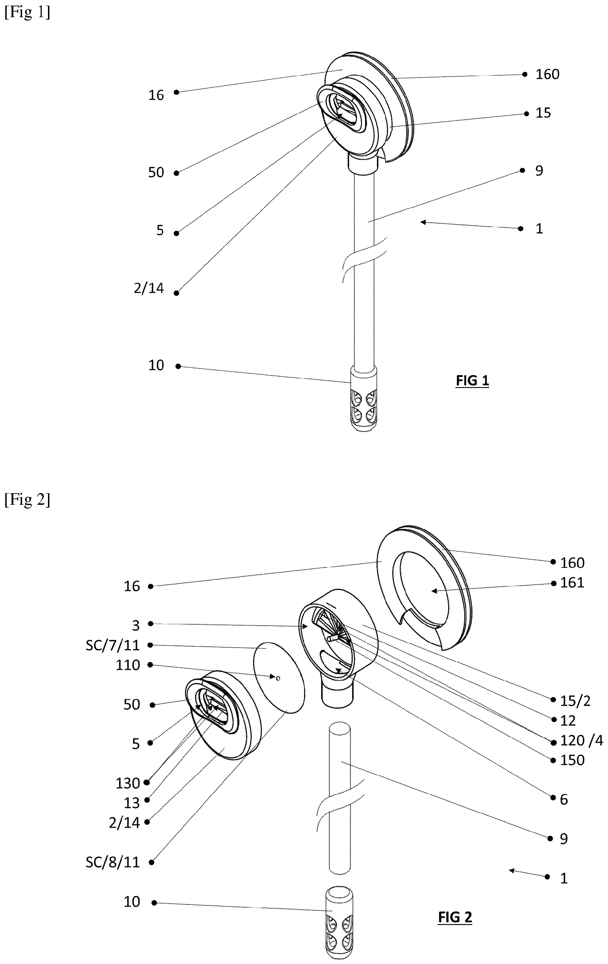

En référence aux figures, le dispositif 1 est considéré en position d'utilisation. Ainsi, la figure 1 présente une vue en perspective du dispositif selon un point de vue arrière droit. Le bas de la figure 1 correspond ainsi au bas du dispositif 1.

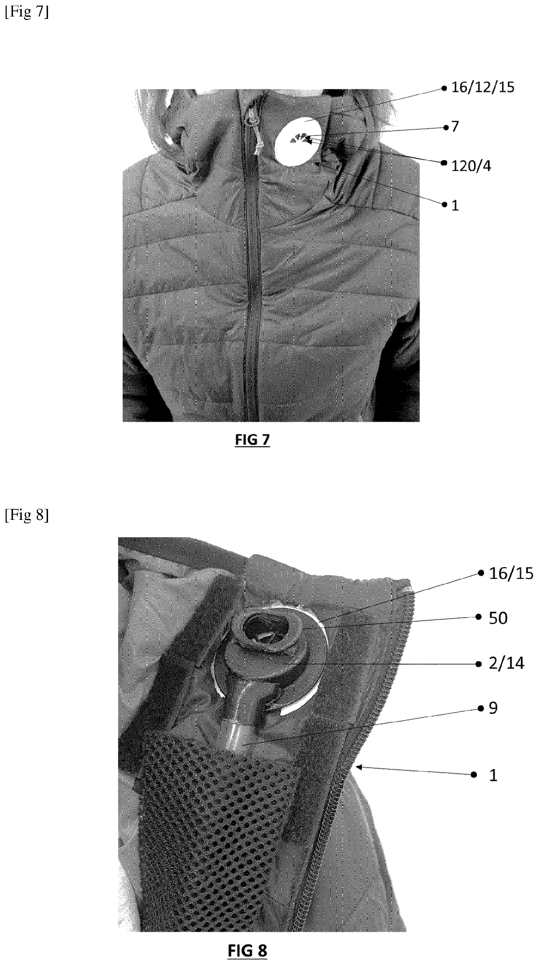

L'arrière du dispositif 1 est visible sur la figure 1 et la figure 8, et l'avant du dispositif est visible sur la figure 7.

Cette chambre 3 reçoit un système de clapets SC comportant au moins trois orifices 4, 5,6 traversant des parois du boitier 2 qui relient l'extérieur dudit boitier 2 à la chambre 3.

Un premier orifice 4 dit orifice d'inspiration 4 traverse une paroi avant du boitier 2, à l'avant du dispositif 1. Ce premier orifice 4 peut être d'un seul tenant ou être scindé en plusieurs jours par une grille ou des barreaux venant de matière avec la paroi.

Un deuxième orifice 5 dit orifice de respiration 5 traverse une paroi arrière du boitier 2, à l'arrière du dispositif 1. L'orifice de respiration 5 peut comporter un embout buccal 50 rapporté ou venant de matière avec le dispositif 1, permettant un maintien du dispositif contre les lèvres ou au moins partiellement dans la bouche d'un utilisateur, comme illustré sur les figures 1, 2 et 8. L'orifice de respiration 5 est configuré pour être mis au moins partiellement dans la bouche ou contre les lèvres d'un utilisateur pour lui permettre d'inspirer et d'expirer de l'air à travers le dispositif 1. Ce deuxième orifice 5 peut être d'un seul tenant, ou être scindé en plusieurs jours par une grille ou des barreaux venant de matière avec la paroi.

Un troisième orifice 6 dit orifice d'expiration 6 traverse une paroi en bas du boitier 2, en bas du dispositif 1. Ce troisième orifice 5 peut être d'un seul tenant ou être scindé en plusieurs jours par une grille ou des barreaux venant de matière avec la paroi.

Le système de clapets SC comprend également au moins un premier clapet 7 et un deuxième clapet 8.

Le premier clapet 7 permet une circulation d'air depuis l'orifice d'inspiration 4 vers l'orifice de respiration 5. Ce premier clapet 7 est anti-retour, c'est-à-dire qu'il bloque la circulation d'air dans le sens opposé.

Le deuxième clapet 8 permet une circulation d'air depuis l'orifice de respiration 5 vers l'orifice d'expiration 6. Ce deuxième clapet 8 est anti-retour, c'est-à-dire qu'il bloque la circulation d'air dans le sens opposé.

Le dispositif comporte également au moins un tube de sortie 9 connecté d'une part à l'orifice d'expiration 6, et d'autre part à un organe de diffusion de la chaleur 10.

Selon l'invention, le premier clapet anti-retour 7 et le deuxième clapet anti-retour 8 sont formées d'une seule pièce 11 au moins partiellement élastiquement déformable.

Cette pièce 11 peut aussi être élastiquement déformable sur toute sa surface.

En fait, la pièce peut comporter au moins deux parties formant respectivement le premier clapet 7 et le deuxième clapet 8.

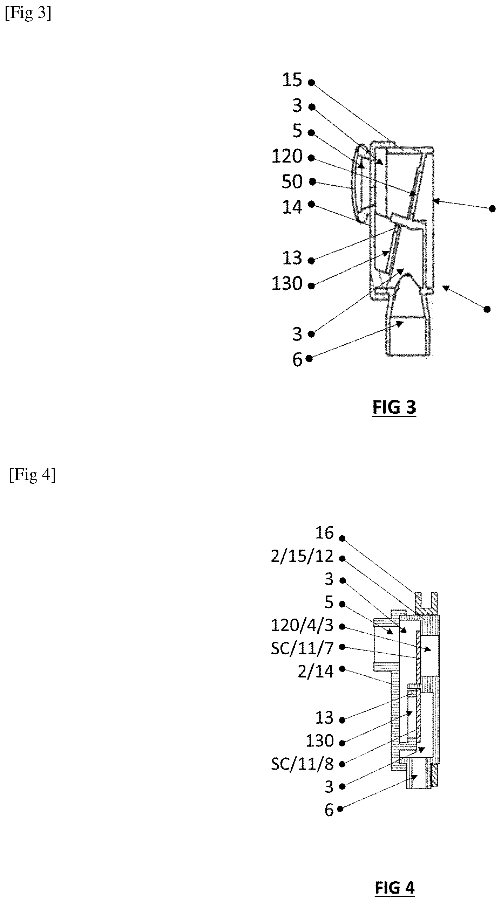

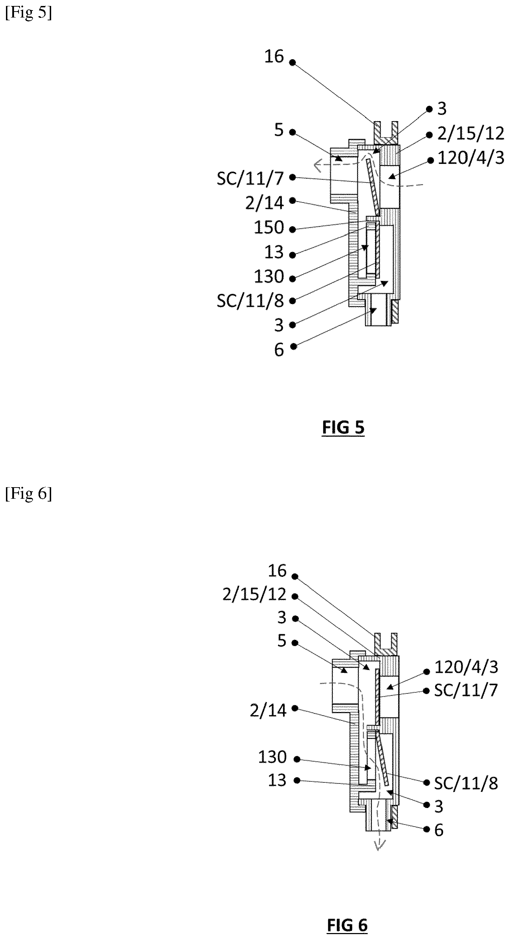

Le premier clapet 7 peut présenter une position de repos, comme illustré sur la figure 4, dans laquelle il bouche la circulation d'air depuis l'orifice d'inspiration 4 vers l'orifice de respiration 5. Le premier clapet 7 peut présenter une position ouverte, comme illustré sur la figure 5.

Cette position ouverte est instable et est obtenue en opposition d'un effet de rappel élastique de la pièce 11 vers la position de repos du premier clapet 7. Cet effort de s'écarter de la position de repos se fait par une action d'aspiration d'air par un effet d'inspiration d'air par un utilisateur à travers le dispositif 1. Une fois que l'aspiration cesse, l'effet de rappel élastique de la pièce 11 ramène le premier clapet 7 à sa position de repos.

Le deuxième clapet 8 peut présenter une position de repos, comme illustré sur la figure 4, dans laquelle il bouche la circulation d'air depuis l'orifice de respiration 5 vers l'orifice d'expiration 6. Le deuxième clapet 8 peut présenter une position ouverte, comme illustré sur la figure 6.

Cette position ouverte est instable et est obtenue en opposition d'un effet de rappel élastique de la pièce 11 vers la position de repos du deuxième clapet 8. Cet effort de s'écarter de la position de repos se fait par une action de soufflage d'air par un effet d'expiration d'air par un utilisateur à travers le dispositif 1. Une fois que l'expiration cesse, l'effet de rappel élastique de la pièce 11 ramène le deuxième clapet 8 à sa position de repos.

Il convient de noter que la figure 5 présente le passage schématique de l'air à travers le dispositif sous la forme d'une flèche en pointillés, avec le premier clapet 7 en position ouverte et le deuxième clapet 8 en position de repos.

Il convient de noter que la figure 6 présente le passage schématique de l'air à travers le dispositif sous la forme d'une flèche en pointillés, avec le deuxième clapet 8 en position ouverte et le premier clapet 7 en position de repos.

La pièce 11 formant à la fois le premier clapet 7 et le deuxième clapet 8 peut être une feuille en un matériau élastomère, comme par exemple en silicone ou en un polymère thermoplastique élastomère tel que par exemple de la famille des Polystyrène Polyéthylène-Butylène, ou de la famille des thermoplastiques copolymères à bloc éther, ester, ou isocyanates.

Toute la surface du ou des clapets 7 et/ou 8 peuvent être élastiquement déformables, ou seulement une zone formant une charnière, pour un rappel élastique du ou des clapets 7 et/ou 8 vers sa ou leurs positions de repos.

Tel qu'illustré sur la figure 2, la pièce 11 peut se présenter sous la forme d'un disque en un matériau élastomère.

Le dispositif 1 comporte une première paroi ajourée dite première paroi d'appui 12, comportant une ouverture 120 ou une pluralité de jours 120 contre laquelle le premier clapet 7 vient en appui en position de repos, de manière à venir recouvrir complètement l'ouverture ou lesdits jours 120 de ladite première paroi d'appui 12 et empêcher lorsque le premier clapet 7 est en position de repos que de l'air passe à travers l'ouverture ou lesdits jours 120.

Le fait que la première paroi d'appui 12 présente une seule ouverture 120 est plus simple à fabriquer, mais une trop large ouverture 120 présente l'inconvénient d'augmenter un risque que le premier clapet 7 ne vienne pas obturer efficacement cette ouverture 120 si elle est trop large et que le bord de l'ouverture 120 présente une surface d'appui du premier clapet 7 trop réduite. Le fait de scinder une ouverture en plusieurs jours 120 par une grille ou des barreaux venant de matière avec la paroi permet d'améliorer l'appui du premier clapet 7 contre la première paroi d'appui 12 et d'avoir une surface de passage d'air plus importante.

La première paroi d'appui 12 se situe à l'avant de la chambre 3, comme illustré sur les figures 2, 4 à 5 et 7.

Dans les exemples de réalisation de l'invention décrits ici, l'orifice d'inspiration 4 est en demi-cercle et scindé en des jours par des barres radiales, il forme les jours 120 dans la première paroi d'appui 12.

Le dispositif 1 comporte également une deuxième paroi ajourée dite deuxième paroi d'appui 13, comportant une ouverture 130 ou une pluralité de jours 130 contre laquelle le deuxième clapet 8 vient en appui en position de repos, de manière à venir recouvrir complètement l'ouverture ou lesdits jours 130 de ladite deuxième paroi d'appui 13 et empêcher lorsque le deuxième clapet 8 est en position de repos que de l'air passe à travers l'ouverture ou lesdits jours 130.

Comme expliqué précédemment, le fait que la deuxième paroi d'appui 13 présente une seule ouverture 130 est plus simple à fabriquer, mais une trop large ouverture 130 présente l'inconvénient d'augmenter un risque que le deuxième clapet 8 ne vienne pas obturer efficacement cette ouverture 130 si elle est trop large et que le bord de l'ouverture 130 présente une surface d'appui du deuxième clapet 8 trop réduite. Le fait de scinder une ouverture en plusieurs jours 130 par une grille ou des barreaux venant de matière avec la paroi permet d'améliorer l'appui du deuxième clapet 8 contre la deuxième paroi d'appui 13 et d'avoir une surface de passage d'air plus importante.

La seconde paroi d'appui 12 se situe dans à l'arrière de la chambre 3, comme illustré sur les figures 4 à 7.

L'ensemble formé du boitier 2 et du système de clapets SC peut être constitué de seulement trois éléments, ce qui réduit les coûts de fabrication et rend le dispositif 1 économique à fabriquer.

Le boitier 2 peut comporter deux demi-coques 14, 15 coopérant entre elle pour former le boitier 2, par exemple par emboitement. Les deux demi-coques 14, 15 peut coopérer également par collage, soudage ou tout autre moyen d'assemblage propre à former le boitier 2 par leur coopération.

Ainsi, une première demi-coque 14 peut former l'arrière du boitier 2, et comporter une paroi arrière avec l'orifice de respiration 5 ainsi que la deuxième paroi ajourée 13, alors qu'une deuxième demi-coque 15 peut former l'avant du boitier 2, et comporter une paroi avant avec l'orifice d'inspiration 4 formant la deuxième paroi ajourée 13.

La deuxième demi-coque 15 peut comporter un doigt central 150 s'étendant vers l'arrière, sur lequel vient se monter la pièce 11 en forme de disque présentant un trou central 110 venant coopérer avec ledit doigt central 150.

L'assemblage des trois éléments se fait en intercalant le disque entre les deux demi-coques 14,15.

Comme illustré sur les figures, le boitier 2 et la pièce 11 peuvent être cylindriques. Les première et deuxième paroi d'appui 12, 13 peuvent être disposés en demi-cercle, avec un coté arrondi et un côté droit comme illustré sur les figures. Les ouvertures ou jours 120, 130 respectifs des première et deuxième parois d'appui 12, 13 peuvent présenter une disposition ou une forme en demi-cercle également comme illustré sur les figures notamment 2, 4 à 8.

En fait, la pièce 11 peut être maintenue dans la chambre 3 par assemblage sur le doigt 150, mais aussi par pincement ou maintien entre la première et la deuxième paroi d'appui 12, 13, plus particulièrement entre leurs côtés droits, comme illustré sur les figures 4 à 6.

La chambre 3 est par cette configuration d'assemblage scindée par la feuille 11 en trois espaces distincts lorsque les clapets sont en position de repos, un espace arrière et deux espace avant.

En fait, ce mode de réalisation de l'invention permet de former un ensemble de boitier 2 et de système de clapet SC compact en peu de pièce, robuste et performant.

Il est ainsi possible de proposer un ensemble de boitier 2 et de système de clapet SC inclus dans un cylindre droit de 33 millimètres de diamètre maximum et 17 millimètres de hauteur maximum, le rendant apte à pouvoir être intégré de manière discrète dans un col d'un vêtement.

La pièce 11 formant à la fois le premier clapet 7 et le deuxième clapet 8 ainsi que les première et deuxième paroi d'appui 12, 13 peuvent être positionnées verticalement comme illustré sur les figures 4 à 6.

Bien entendu, le boitier 2 peut comporter des première et deuxième paroi d'appui 12, 13 légèrement oblique plutôt que verticales, comme illustré sur la figure 3, pour permettre une amplitude de mouvement plus importante de la pièce 11 et augmenter la surface de passage des flux d'air à travers le dispositif et le système de clapet SC.

Il est évident que la forme du boitier 2 et la disposition des clapets 7,8 et des orifices 4, 5, 6 sur le boitier 2 peuvent varier. Ainsi, par exemple, le boitier 2 peut être parallélépipédique, et/ou les clapets 7,8 peuvent être juxtaposés horizontalement, pour une intégration horizontale dans un col de vêtement.

Le dispositif 1, notamment la chambre 3, les orifices 4,5,6 et le système de clapet SC sont configurés pour que la section de passage d'air entre l'orifice d'inspiration et l'orifice de respiration et la section de passage d'air entre l'orifice de respiration et l'organe de diffusion soit supérieure à 75 millimètres carrés.

Cela permet à un utilisateur de respirer confortablement lors de l'inspiration et de l'expiration à travers le boitier 2.

Le tube de sortie 9 du dispositif peut être connecté par emboitement à l'orifice d'expiration 6 ou tout autre moyen d'assemblage connu tel que collage, vissage ou montage en force. Le tuyau de sortie peut aussi venir de matière avec l'une et/ou l'autre des demi-coques 14,15 du boitier 2. Il peut être extrudé en un matériau thermoplastique tel que par exemple en Polypropylène ou en Polychlorure de Vinyle.

Le tube de sortie 9 présente ici une longueur de quelques dizaines de centimètres et un diamètre intérieur de 10 millimètres, soit 78 millimètre carrés à un millimètre carré près.

Il permet d'envoyer l'air expiré vers une zone du corps autre que le col du vêtement, comme par exemple vers le torse, la poitrine, le ventre, les flancs.

Il a été observé que la longueur du tube de sortie 9 avait peu d'incidence sur le confort d'utilisation du dispositif 1, qu'il fasse dix centimètre ou quarante centimètres.

Un diamètre intérieur de 10 millimètres et une longueur de tube de sortie 9 inférieur à 40 centimètres n'apporte pas de contrainte significative sur l'expiration.

Des tests réalisés ont montré que la longueur du tube modifiait peu la chaleur délivrée du moment que le boitier soit au niveau du col du vêtement. Des mesures en caméra thermique montrent une diffusion dans tout l'espace situé entre le vêtement et le sujet.

En fait, le dispositif peut comporter un tube de sortie de quelques centimètres seulement, et l'organe de diffusion de chaleur peut n'être qu'une ouverture vers le bas du tube de sortie 9, car le fait que le flux d'air chaud soit dirigé vers le bas permet déjà une bonne diffusion de la chaleur.

L'organe de diffusion de chaleur 10 peut se présenter sous la forme d'un cylindre ouvert à une extrémité dans lequel peut s'emboiter le tube de sortie, et comportant une pluralité de trous latéraux. Ces trous latéraux peuvent présenter une surface totale supérieure à 75 millimètre carrés, voire supérieure à 100 millimètre carré, pour assurer un confort d'utilisation lors de l'expiration d'un utilisateur. Le cylindre peut comporter à son autre extrémité des moyens de rétention de la condensation de l'eau présente dans l'air expiré, par exemple sous la forme d'une éponge ou d'un réservoir.

L'organe de diffusion de chaleur peut également se présenter sous la forme d'un poche textile perméable à l'air ou sous la forme d'un réseau de canaux percés ou non sur leur longueur et débouchant à leur extrémité, ce réseau couvrant une partie du corps de l'utilisateur.

Le dispositif peut comporter également un anneau d'assemblage 16 configuré pour être placé sur un col d'un vêtement. Cet anneau d'assemblage 16 peut comporter une gorge 160 sur tout ou partie de sa périphérie, permettant l'insertion du rebord d'un trou dans un col de vêtement. Une fois le rebord inséré dans la gorge, il peut être collé, ou solidarisé avec les parois de la gorge par tout autre moyen d'assemblage réversible ou non.

Il est ainsi aisé de percer un trou dans un col de vêtement pour y placer l'anneau d'assemblage 16, qui reste discret et compact.

Cet anneau d'assemblage 16 peut comporter une ouverture centrale 161 configurée pour recevoir le boitier 2. Des moyens de maintien du boitier 2 dans l'ouverture centrale 161 peuvent être prévus, comme par exemple un emboitement monté serré ou un filetage de l'ouverture centrale 161 et un pas de vis sur la périphérie du boitier 2.

Dans le mode de réalisation de l'invention illustré sur les figures 7 et 8, l'anneau d'assemblage 16 est venu de matière avec la deuxième coque 15.

Le dispositif 1 est compact et discret du fait de sa configuration particulière. Il peut être intégré simplement et facilement dans un vêtement, avec un ensemble de boitier 2 et le système de clapet SC plat et présentant une grande liberté de forme possible pour une intégration aisée dans un vêtement et plus particulièrement un col de vêtement.

Il est possible selon un mode de réalisation alternatif de prévoir un embout buccal 50 plus long, d'une taille comprise entre deux centimètre et une dizaine de centimètres, ce qui permet de positionner l'ensemble de boitier 2 et le système de clapet SC un peu plus bas qu'au niveau du col d'un vêtement, pour conserver le confort du vêtement même équipé du dispositif 1. L'embout buccal 50 présente alors une section de passage d'air supérieure ou égale à 75 millimètre carré pour ne pas obérer la respiration via ce dispositif 1.

L'invention concerne également un vêtement comportant un dispositif de chauffage corporel présentant les caractéristiques du dispositif 1 décrit ci avant.

Ce vêtement peut également comporter des moyens de maintien du tube de sortie 9 et/ou de l'organe de diffusion 10, sous la forme par exemple d'une poche présentant une face interne paroi en maille résille, comme illustré sur la figure 8, ou d'un canal dans la doublure dudit vêtement pouvant recevoir le tube de sortie 9 ouvert à son extrémité pour laisser sortir l'organe de diffusion 10.

## Revendications

1. Dispositif de chauffage corporel (1) utilisant l'air expiré d'un utilisateur équipé d'un tel dispositif (1), ledit dispositif (1) comportant au moins :
- un boitier (2) délimitant une chambre (3) avec un système de clapets (SC) comprenant au moins trois orifices (4,5,6) connectant l'extérieur dudit boitier (2) à ladite chambre (3), lesdits au moins trois orifices (4,5,6) comportant un premier orifice (4) dit d'inspiration, un deuxième orifice (5) dit de respiration et un troisième orifice (6) dit d'expiration, ledit système de clapets (SC) comprenant un premier clapet (7) qui permet la circulation d'air de l'orifice d'inspiration (4) vers l'orifice de respiration (5), et un deuxième clapet (8) qui permet la circulation d'air de l'orifice de respiration (5) vers l'orifice d'expiration (6),
- un tube de sortie (9) connecté d'une part à l'orifice d'expiration (6) et d'autre part à un organe de diffusion (10) de la chaleur,
**caractérisé en ce que** le premier clapet (7) et le deuxième clapet (8) sont formées d'une seule pièce (11) au moins partiellement élastiquement déformable et **en ce que** l'ensemble formé du boitier (2) avec la chambre (3) et du système de clapets (SC) est constitué de trois éléments constitués d'une part de deux demi-coques (14, 15) coopérant l'une avec l'autre par emboitement, et d'autre part de la pièce (11) formant le premier clapet (7) et le deuxième clapet (8) intercalée entre les deux demi-coques (14, 15).

2. Dispositif (1) selon la revendication 1, **caractérisé par le fait que** la pièce (11) au moins partiellement élastiquement déformable est une feuille en un matériau élastomère.

3. Dispositif (1) selon l'une des revendication 1 ou 2, **caractérisé par le fait que** le boitier (2) du dispositif (1) comporte deux parois (12, 13) ajourées contre lesquelles des parties de la pièce (11) formant le premier clapet (7) et le deuxième clapet (8) viennent en appui.

4. Dispositif (1) selon l'une des revendications précédentes , **caractérisé en ce que** l'ensemble formé du boitier (2) avec la chambre (3) et du système de clapets (SC) est inclus dans un cylindre droit de 33 millimètres de diamètre maximum et de 17 millimètres de hauteur maximum.

5. Dispositif (1) selon l'une des revendications précédentes, **caractérisé par le fait que** la section de passage d'air entre l'orifice d'inspiration (4) et l'orifice de respiration (5), et la section de passage d'air entre l'orifice de respiration (5) et l'organe de diffusion (6) est supérieure à 75 millimètres carrés.

6. Dispositif (1) selon l'une des revendications précédentes, **caractérisé en ce que** l'organe de diffusion (10) connecté au tube de sortie (9) est un cylindre présentant une pluralité de trous latéraux.

7. Dispositif (1) selon l'une des revendications précédentes, **caractérisé par le fait que** le dispositif comporte un anneau d'assemblage (16), destiné à être placé sur un col de vêtement, ledit anneau d'assemblage (16) comportant une ouverture centrale (161) configurée pour recevoir le boitier (2).

8. Vêtement comportant un dispositif (1) selon la revendication précédente, ledit vêtement comportant également des moyens de maintien du tube de sortie et/ou de l'organe de diffusion.
